# EUROPÄISCHE PATENTSCHRIFT

(11) **EP 2 588 442 B1**
(45) Veröffentlichungstag und Bekanntmachung des Hinweises auf die Patenterteilung: **16.04.2014**
(21) Anmeldenummer: 11730932.8
(22) Anmeldetag: 28.06.2011
(51) Int. Cl.: C07C 209/64, C07C 211/06, C07C 209/16, B01J 23/72, B01J 23/755

(54) **VERFAHREN ZUR HERSTELLUNG VON TRI-N-PROPYLAMIN (TPA)**
METHOD FOR PRODUCING TRI-N-PROPYL AMINE (TPA)
PROCÉDÉ DE PRODUCTION DE TRI-N-PROPYLAMINE (TPA)

(30) Priorität: 30.06.2010 EP 10167916
(43) Veröffentlichungstag der Anmeldung: 08.05.2013
(73) Patentinhaber: BASF SE, 67056 Ludwigshafen (DE)
(72) Erfinder: HUYGHE, Kevin, B-2950 Kapellen (BE); BRUGHMANS, Steven, 68167 Mannheim (DE); SIMON, Falk, 64625 Bensheim (DE); MELDER, Johann-Peter, 67459 Böhl-Iggelheim (DE); RAATZ, Peter, 67069 Ludwigshafen (DE)
(86) Internationale Anmeldenummer: PCT/EP2011/060755
(87) Internationale Veröffentlichungsnummer: WO 2012/000952

(56) Entgegenhaltungen:
- EP-A1- 0 379 939

## Beschreibung

Die Erfindung betrifft ein Verfahren zur Herstellung von Tri-n-propylamin (TPA).

Tri-n-propylamin (TPA) ist u.a. ein wichtiges Zwischenprodukt zur Herstellung von Farbstoffen, Katalysatoren und Korrosionsinhibitoren und zur Anwendung in der Pharma- und Kosmetik-Industrie (vgl. z. B. BASF Technical Data Sheet ,Tripropylamine').

Bei der Aminierung von n-Propanol mit Ammoniak wird immer ein Produktgemisch aus Mono-n-propylamin (MPA), Di-n-propylamin (DPA) und Tri-n-propylamin (TPA) erhalten. Das entstehende Amin-Produktgemisch kann in seiner Zusammensetzung großenteils über die Prozessparameter bei der Umsetzung des Propanols gesteuert werden. Allerdings ist vor allem die anteilige Di-n-propylaminmenge im Produktgemisch schwierig zu beeinflussen und ist es oft unmöglich genau das vom Markt gefragte Amin-Produktgemisch herzustellen.

Eine Möglichkeit der Steuerung des Amin-Produktgemisches wäre die separate Umsetzung vom Dialkylamin zum Trialkylamin durch Reaktion mit dem n-Propanol. Allerdings ist diese Möglichkeit aus prozesstechnischen und -chemischen Gründen nicht bevorzugt [Gefahr von sog. Durchgehreaktionen (unkontrollierter Temperaturanstieg), Sicherheitsaspekt].

Aus NL 65644 und dem äquivalenten US 2,574,693 (Shell Dev. Comp.) ist bekannt, dass man Monobutylamin zu Dibutylamin an einem Al₂O₃-Katalysator in Gegenwart von Ammoniak bei hohen Temperaturen umsetzen kann.

CN 1,325,842 A (Chinese Petro. Chem. Group) lehrt die Umsetzung von Monoisopropylamin zu Diisopropylamin an einem K/H-beta-Zeolithen bei erhöhter Temperatur.

EP 379 939 A1 offenbart ein Verfahren zur Herstellung von Trialkylaminen durch Umsetzung von Dialkylaminen mit einem Alkanol in Gegenwart von Wasserstoff und eines Hydrierungs-/Dehydrierungskatalysators.

Der vorliegenden Erfindung lag die Aufgabe zugrunde, unter Überwindung von Nachteilen des Stands der Technik, ein verbessertes wirtschaftliches Verfahren zur Herstellung von Tri-n-propylamin (TPA) bereitzustellen. Das Herstellverfahren sollte von Tri-n-propylamin in hoher Ausbeute, Raumzeitausbeute (RZA) und Selektivität liefern und zudem besonders einfach und wirtschaftlich sein.

Erfindungsgemäß wurde erkannt, dass durch eine Umsetzung (Scrambling) von Di-n-propylamin, optional in Anwesenheit von Ammoniak, bevorzugt in Abwesenheit von Ammoniak, das, z. B. durch vorhergehende Aminierung von n-Propanol entstandene, Di-n-propylamin zu Tri-n-propylamin umgewandelt und damit der n-Propylamin-Produktmix einer vorhergehenden n-Propylaminsynthese gezielt zugunsten des tertiären Amins verändert werden kann. Demgemäß wurde ein Verfahren zur Herstellung von Tri-n-propylamin gefunden, welches dadurch gekennzeichnet ist, dass man Di-n-Propylamin in Gegenwart von Wasserstoff und eines kupferhaltigen Heterogen-Katalysators umsetzt.
Die Reaktion des DPAs erfolgt nach dem Schema

Di-n-propylamin ---> Tri-n-propylamin + NH₃

Als Nebenprodukt fallen geringe Mengen an Mono-n-propylamin an.

Bei der Disproportionierung wird das Di-n-propylamin an einem kupferhaltigen Heterogen-Katalysator umgesetzt. Bevorzugt erhöhter Druck, bevorzugt erhöhte Temperatur und die Anwesenheit von Wasserstoff sind die typischen Reaktionsbedingungen.

Bevorzugt wird die Umsetzung bei einem Absolutdruck im Bereich von 20 bis 150 bar, besonders 40 bis 150 bar, weiter besonders 60 bis 150 bar, durchgeführt.

Die Umsetzung erfolgt bevorzugt bei einer Temperatur im Bereich von 180 bis 260 °C, besonders 190 bis 260 °C, weiter besonders 200 bis 260 °C.

Die Katalysatorbelastung liegt bevorzugt im Bereich von 0,3 bis 3 kg/l•h, besonders 0,3 bis 0,7 kg/l•h, weiter besonders 0,4 bis 0,7 kg/l•h [kg DPA / (Liter Katalysator Stunde)]. (Liter Katalysator = Katalysatorschüttvolumen)

Die eingesetzte Wasserstoffmenge liegt bevorzugt im Bereich von 200 bis 2000 Nl/l•h, besonders 250 bis 700 Nl/l•h, weiter besonders 300 bis 600 Nl/l•h [Normliter / (Liter Katalysator Stunde)]
(Nl = Normliter = auf Normalbedingungen (20 °C, 1 bar absolut) umgerechnetes Volumen).

Das Verfahren kann kontinuierlich oder diskontinierlich durchgeführt werden. Bevorzugt ist eine kontinuierliche Fahrweise.

Für die Synthese wird das Edukt (DPA) bevorzugt in einem Wasserstoffstrom aufgeheizt und dem Reaktor zugeführt. Wasserstoff wird bevorzugt in einer Kreisgasfahrweise gefahren.

Das Edukt (DPA) kann gegebenenfalls aus einer Destillationskolonne, in der Reaktionsaustrag aufgetrennt wurde, rückgeführt werden.

Das Edukt (DPA) kann auch als wässrige Lösung aufgeheizt und, bevorzugt mit dem Kreisgasstrom, auf das Katalysatorbett geleitet werden.

Bevorzugte Reaktoren sind Rohrreaktoren. Beispiele für geeignete Reaktoren mit Kreisgasstrom finden sich in Ullmann's Encyclopedia of Industrial Chemistry, 5th Ed., Vol. B 4, Seiten 199-238, "Fixed-Bed Reactors".

Alternativ erfolgt die Umsetzung vorteilhaft in einem Rohrbündelreaktor oder in einer Monostranganlage.

Bei einer Monostranganlage kann der Rohrreaktor, in dem die Umsetzung erfolgt, aus einer Hintereinanderschaltung mehrerer (z. B. zweier oder dreier) einzelner Rohrreaktoren bestehen. Optional ist hier vorteilhaft eine Zwischeneinspeisung von Feed (enthaltend das Edukt DPA und/oder H₂) und/oder Kreisgas und/oder Reaktoraustrag aus einem nachgeschalteten Reaktor möglich.

Der im erfindungsgemäßen Verfahren eingesetzte Heterogen-Katalysator enthält Cu, bevorzugt Cu und Ni und/oder Co, besonders bevorzugt Cu und Ni und Co.

Bevorzugt enthält der Heterogen-Katalysator ein oxidisches Trägermaterial für die Aktivmetalle, bevorzugt Aluminiumoxid (gamma, delta, theta, alpha, kappa, chi oder Mischungen daraus) und/oder Zirkoniumdioxid (bevorzugt monokline, tetragonale oder kubische Modifikation). Besonders bevorzugtes Trägermaterial ist Aluminiumoxid, insbesondere gamma-Aluminiumoxid.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.
In diesem Zusammenhang wird das oxidische Trägermaterial Aluminiumoxid (Al₂O₃), Zirkoniumdioxid (ZrO₂) als zur katalytisch aktiven Masse gehörig gewertet.

Die Katalysatoren werden dergestalt eingesetzt, dass man die katalytisch aktive, zu Pulver vermahlene Masse in das Reaktionsgefäß einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im wesentlichen die folgenden Bestandteile:
Aluminiumoxid (Al₂O₃) und/oder Zirkoniumdioxid (ZrO₂) und sauerstoffhaltige Verbindungen des Kupfers und/oder Nickels und/oder Kobalts.

Die Summe der o. g. Bestandteile der katalytisch aktiven Masse beträgt üblicherweise 70 bis 100 Gew.-%, bevorzugt 80 bis 100 Gew.-%, besonders bevorzugt 90 bis 100 Gew.-%, besonders > 95 Gew.-%, ganz besonders > 98 Gew.-%, insbesondere > 99 Gew.-%, z. B. besonders bevorzugt 100 Gew.-%.

Die katalytisch aktive Masse der erfindungsgemäßen und im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:
Übergangsmetalle, wie Mn bzw. MnO₂, Mo bzw. MoO₃, W bzw. Wolframoxide, Ta bzw. Tantaloxide, Nb bzw. Nioboxide oder Nioboxalat, V bzw. Vanadiumoxide bzw. Vanadylpyrophosphat;
Lanthanide, wie Ce bzw. CeO₂ oder Pr bzw. Pr₂O₃; Erdalkalimetalloxide, wie SrO; Erdalkalimetallcarbonate, wie MgCO₃, CaCO₃ und BaCO₃; Alkalimetalloxide, wie Na₂O, K₂O; Alkalimetall-carbonate, wie Li₂CO₃, Na₂CO₃ und K₂CO₃; Boroxid (B₂O₃).

Bevorzugt enthält die katalytisch aktive Masse des im erfindungsgemäßen Verfahren eingesetzten Katalysators kein Rhenium, kein Ruthenium, kein Eisen und/oder kein Zink, jeweils weder in metallischer (Oxidationsstufe = 0) noch in einer ionischen (Oxidationsstufe ≠ 0), insbesondere oxidierten, Form.

Bevorzugt enthält die katalytisch aktive Masse des im erfindungsgemäßen Verfahren eingesetzten Katalysators kein Silber und/oder Molybdän, jeweils weder in metallischer (Oxidationsstufe = 0) noch in einer ionischen (Oxidationsstufe ≠ 0), insbesondere oxidierten, Form.

Bevorzugt enthält die katalytisch aktive Masse des Katalysators keine sauerstoffhaltigen Verbindungen des Siliziums und/oder des Chroms.

Die Katalysatoren können nach bekannten Verfahren, z. B. durch Fällung, Auffällung, Imprägnierung, hergestellt werden.

Bevorzugte Heterogen-Katalysatoren enthalten in ihrer katalytisch aktiven Masse vor der Behandlung mit Wasserstoff

20 bis 85 Gew.-%, bevorzugt 20 bis 65 Gew.-%, besonders bevorzugt 22 bis 40 Gew.-%, sauerstoffhaltige Verbindungen des Zirkoniums, berechnet als ZrO₂,

1 bis 30 Gew.-%, besonders bevorzugt 2 bis 25 Gew.-%, sauerstoffhaltige Verbindungen des Kupfers, berechnet als CuO,

14 bis 70 Gew.-%, bevorzugt 15 bis 50 Gew.-%, besonders bevorzugt 21 bis 45 Gew.-%, sauerstoffhaltige Verbindungen des Nickels, berechnet als NiO, wobei bevorzugt das Molverhältnis von Nickel zu Kupfer größer 1, insbesondere größer 1,2, ganz besonders 1,8 bis 8,5, ist, und

0 bis 5 Gew.-%, besonders 0,1 bis 3 Gew.-%, sauerstoffhaltige Verbindungen des Molybdäns, berechnet als MoO₃.

In einer weiteren Variante enthalten diese bevorzugten Katalysatoren zusätzlich in ihrer katalytisch aktiven Masse vor der Behandlung mit Wasserstoff

15 bis 50 Gew.-%, besonders bevorzugt 21 bis 45 Gew.-%, sauerstoffhaltige Verbindungen des Kobalts, berechnet als CoO.

Die sauerstoffhaltigen Verbindungen des Kupfers, Nickels und gegebenenfalls Kobalts, jeweils berechnet als CuO, NiO und CoO, der bevorzugten Katalysatoren sind im allgemeinen insgesamt in Mengen von 15 bis 80 Gew.-%, bevorzugt 35 bis 80 Gew.-%, besonders bevorzugt 60 bis 78 Gew.-%, in der katalytisch aktiven Masse (vor der Behandlung mit Wasserstoff) enthalten, wobei besonders bevorzugt das Molverhältnis von Nickel zu Kupfer größer 1 ist.

Besonders bevorzugte Heterogen-Katalysatoren enthalten in ihrer katalytisch aktiven Masse vor der Behandlung mit Wasserstoff
20 bis 90 Gew.-%, bevorzugt 40 bis 85 Gew.-%, besonders bevorzugt 60 bis 80 Gew.-%, sauerstoffhaltige Verbindungen des Aluminiums, berechnet als Al₂O₃,
1 bis 30 Gew.-%, bevorzugt 2 bis 25 Gew.-%, besonders bevorzugt 3 bis 20 Gew.-%, sauerstoffhaltige Verbindungen des Kupfers, berechnet als CuO,
1 bis 40 Gew.-%, bevorzugt 3 bis 30 Gew.-%, besonders bevorzugt 5 bis 20 Gew.-%, sauerstoffhaltige Verbindungen des Nickels, berechnet als NiO, wobei besonders bevorzugt das Molverhältnis von Nickel zu Kupfer größer 1, bevorzugt größer 1,2, besonders bevorzugt 1,8 bis 8,5, ist, und
1 bis 40 Gew.-%, bevorzugt 3 bis 30 Gew.-%, besonders bevorzugt 5 bis 20 Gew.-%, sauerstoffhaltige Verbindungen des Kobalts, berechnet als CoO.

Die sauerstoffhaltigen Verbindungen des Nickels, Kobalts und Kupfers, jeweils berechnet als NiO, CoO und CuO, sind bevorzugt insgesamt in Mengen von 10 bis 80 Gew.-%, besonders bevorzugt 15 bis 60 Gew.-%, ganz besonders bevorzugt 20 bis 40 Gew.-%, in der katalytisch aktiven Masse (vor der Behandlung mit Wasserstoff) enthalten, wobei besonders bevorzugt das Molverhältnis von Nickel zu Kupfer größer 1 ist.

Weitere bevorzugte Heterogenkatalysatoren im erfindungsgemäßen Verfahren sind
in DE 19 53 263 A (BASF AG) offenbarte Katalysatoren enthaltend Kobalt, Nickel und Kupfer und Aluminiumoxid und/oder Siliciumdioxid mit einem Metallgehalt von 5 bis 80 Gew.-%, insbesondere 10 bis 30 Gew.-%, bezogen auf den gesamten Katalysator, wobei die Katalysatoren, berechnet auf den Metallgehalt, 70 bis 95 Gew.-% einer Mischung aus Kobalt und Nickel und 5 bis 30 Gew.-% Kupfer enthalten und wobei das Gewichtsverhältnis von Kobalt zu Nickel 4 : 1 bis 1 : 4, insbesondere 2 : 1 bis 1 : 2, beträgt, beispielsweise der in den dortigen Beispielen verwendete Katalysator mit der Zusammensetzung 10 Gew.-% CoO, 10 Gew.-% NiO und 4 Gew.-% CuO auf Al₂O₃,
in EP 382 049 A (BASF AG) offenbarte bzw. entsprechend herstellbare Katalysatoren, deren katalytisch aktive Masse vor der Behandlung mit Wasserstoff
20 bis 85 Gew.-%, bevorzugt 70 bis 80 Gew.-%, ZrO₂ und/oder Al₂O₃,
1 bis 30 Gew.-%, bevorzugt 1 bis 10 Gew.-%, CuO,
und jeweils 1 bis 40 Gew.-%, bevorzugt 5 bis 20 Gew.-%, CoO und NiO enthält, beispielsweise die in loc. cit. auf Seite 6 beschriebenen Katalysatoren mit der Zusammensetzung 76 Gew.-% Zr, berechnet als ZrO₂, 4 Gew.-% Cu, berechnet als CuO, 10 Gew.-% Co, berechnet als CoO, und 10 Gew.-% Ni, berechnet als NiO,
in EP 963 975 A (BASF AG) offenbarte Katalysatoren, deren katalytisch aktive Masse vor der Behandlung mit Wasserstoff
22 bis 40 Gew.-% ZrO₂,
1 bis 30 Gew.-% sauerstoffhaltige Verbindungen des Kupfers, berechnet als CuO,
15 bis 50 Gew.-% sauerstoffhaltige Verbindungen des Nickels, berechnet als NiO, wobei das molare Ni : Cu-Verhältnis größer 1 ist,
15 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, berechnet als Al₂O₃ bzw. MnO₂,
und keine sauerstoffhaltigen Verbindungen des Molybdäns enthält,
beispielsweise der in loc. cit., Seite 17, offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO,
in EP 696 572 A (BASF AG) offenbarte Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂, enthält, beispielsweise der in loc. cit., Seite 8, offenbarte Katalysator mit der Zusammensetzung 31,5 Gew.-% ZrO₂, 50 Gew.-% NiO, 17 Gew.-% CuO und 1,5 Gew.-% MoO₃,
in EP 1 270 543 A1(BASF AG) beschriebene Katalysatoren, enthaltend mindestens ein Element oder eine Verbindung eines Elementes aus den Gruppen VIII und IB des Periodensystems,
und
in EP 1 431 273 A (BASF AG) beschriebene Katalysatoren, bei deren Herstellung eine Fällung von katalytisch aktiven Komponenten auf monoklines, tetragonales oder kubisches Zirkoniumdioxid erfolgte.

Die hergestellten Katalysatoren können als solche gelagert werden. Vor ihrem Einsatz als Katalysatoren im erfindungsgemäßen Verfahren werden sie durch Behandlung mit Wasserstoff vorreduziert (= Aktivierung des Katalysators). Sie können jedoch auch ohne Vorreduktion eingesetzt werden, wobei sie dann unter den Bedingungen des erfindungsgemäßen Verfahrens durch den im Reaktor vorhandenen Wasserstoff reduziert (= aktiviert) werden.

Zur Aktivierung wird der Katalysator bevorzugt bei einer Temperatur im Bereich von 100 bis 500 °C, besonders 150 bis 400 °C, ganz besonders 180 bis 300 °C, über einen Zeitraum von mindestens 25 Min., besonders mindestens 60 Min., einer Wasserstoff-haltigen Atmosphäre oder einer Wasserstoff-Atmosphäre ausgesetzt. Der Zeitraum der Aktivierung des Katalysators kann bis zu 1 h, besonders bis zu 12 h, insbesondere bis zu 24 h, betragen.

Bei dieser Aktivierung wird zumindest ein Teil der in den Katalysatoren vorliegenden sauerstoffhaltigen Metallverbindungen zu den entsprechenden Metallen reduziert, so dass diese gemeinsam mit den verschiedenartigen Sauerstoffverbindungen in der aktiven Form des Katalysators vorliegen.

Erfindungsgemäß wird im Besonderen das erfindungsgemäße Verfahren zur Herstellung von TPA durch die oben beschriebene Disproportionierung von Di-n-propylamin in einen integrierten Prozess durchgeführt, in dem wie im Folgenden beschrieben insbesondere Tri-n-propylamin (TPA) selektiv hergestellt wird.
Das integrierte Verfahren ermöglicht die genaue Steuerung der Anteile im Amin-Produktgemisch Mono-n-propylamin (MPA), Di-n-propylamin (DPA) und Tri-n-propylamin (TPA). Mit dieser hohen Produktflexibilität kann vorteilhafterweise der vom Markt gefragte Produktmix genau hergestellt werden.

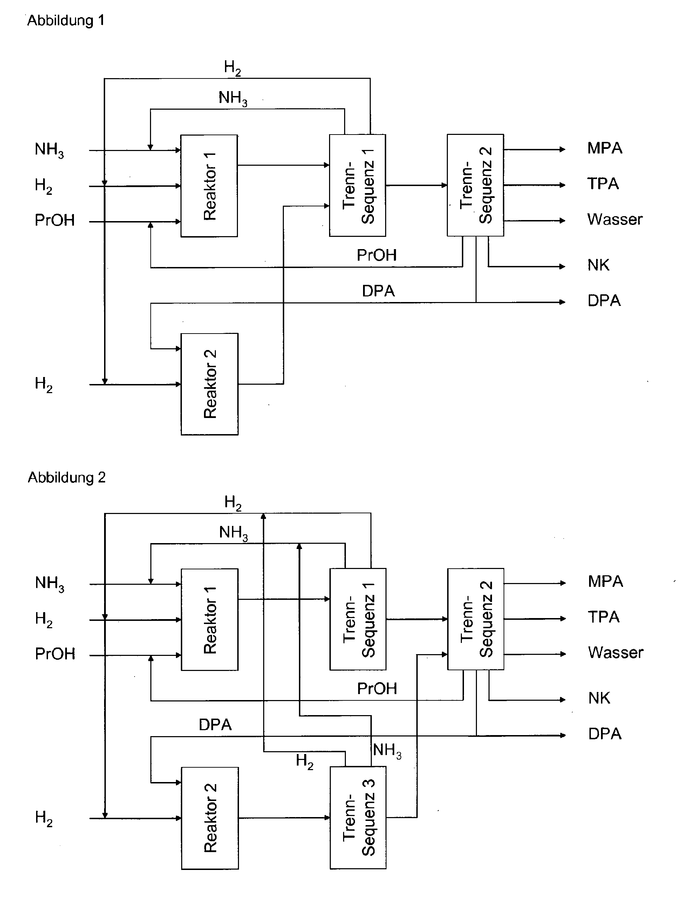

Vergl. die Abbildungen 1 und 2 für bevorzugte Ausführungsformen.

In einem Hauptreaktor findet, bevorzugt kontinuierlich, eine n-Propanolaminierung statt. Dazu wird n-Propanol (PrOH) mit Ammoniak an einem Aminierungskatalysator und gegebenenfalls in Anwesenheit von Wasserstoff zu einem Gemisch von Mono-n-propylamin, Di-n-propylamin und Tri-n-propylamin umgesetzt. Die Umsetzung von n-Propanol mit Ammoniak im Hauptreaktor (Reaktor 1), der natürlich auch in zwei oder mehr seriell oder parallel geschaltete Reaktoren aufgeteilt sein kann, kann nach dem Fachmann bekannten Verfahren durchgeführt werden (siehe z. B. Kirk-Othmer Encyclopedia of Chemical Technology, Vol. 2, Seiten 537-553).

Ammoniak, n-Propanol und gegebenenfalls Wasserstoff werden aus dem Reaktionsaustrag abgetrennt und davon zumindest Ammoniak und Propanol in den Reaktor zurückgeführt. Auch Mono-n-propylamin, Di-n-propylamin und Tri-n-propylamin werden destillativ, z. B. in einer Destillationskolonnenkaskade, getrennt.

Abgetrenntes Di-n-propylamin wird in einen Reaktor (Konvertierungsreaktor) gefahren, in dem erfindungsgemäß in Gegenwart eines kupferhaltigen Heterogen-Katalysators, bevorzugt kontinuierlich, die Umsetzung zu TPA erfolgt.

Der Austrag des Konvertierungsreaktors wird in den o.g. Aufarbeitungsteil des Reaktionsaustrags des Hauptreaktors gefahren.

Mit einer solchen integrierten Fahrweise lässt sich aus n-Propanol mit einer hohen Selektivität, z. B. im Bereich von 40 bis 99 % (bez. auf n-Propanol), Tri-n-propylamin herstellen.

Besondere und bevorzugte Verfahrensausgestaltungen ergeben sich wie folgt:
Die Umsetzung von n-Propanol mit Ammoniak wird in einem Reaktor (Reaktor 1) bevorzugt an einem Übergangsmetallkatalysator, z. B. einem kupfer- und/oder nickelhaltigen Katalysator, bei im allgemeinen 1 bis 220 bar und im allgemeinen 130 bis 250 °C, oder an einem sauren Katalysator, wie z. B. einem Metalloxid oder Zeolith, bei im allgemeinen 1 bis 36 bar und im allgemeinen 300 bis 500 °C, durchgeführt.
Der Katalysator wird im Reaktor (Reaktor 1) bevorzugt als Festbett angeordnet.
Der Propanol-Umsatz wird im allgemeinen bei > 90 %, bevorzugt > 95 %, gehalten.
Zur Aufrechterhaltung der Katalysatoraktivität wird bei Einsatz von Metallkatalysatoren bevorzugt auch Wasserstoff in den Reaktor (Reaktor 1) gefahren.

Der Austrag aus dem Reaktor der n-Propanol-Umsetzung (Reaktor 1) wird anschließend auf bevorzugt 20 bis 30 bar entspannt. Der dabei ggf. anfallende, ggf. noch etwas Ammoniak enthaltende, "Niederdruckwasserstoff` kann verdichtet und gekreist werden über den Reaktor der n-Propanol-Umsetzung (Reaktor 1) und/oder er kann, gegebenenfalls nach seiner Verdichtung, direkt (oder nach der Abtrennung von enthaltenen Ammoniak über eine Gaswäsche) als Feed für den Reaktor zur DPA-Umsetzung (Reaktor 2) (siehe unten) verwendet werden.

Der nach Abtrennung des Wasserstoffs verbleibende Reaktionsaustrag, enthaltend in Wesentlichen oder bestehend aus Ammoniak, Wasser, n-Propanol, MPA, DPA und TPA, wird den unterschiedlichen Dampfdrucken entsprechend in die einzelnen Bestandteile aufgetrennt.

Die mehrstufige Auftrennung in die Bestandteile erfolgt bevorzugt destillativ, insbesondere durch kontinuierliche Destillation und/oder durch flüssig-flüssig Phasentrennung, insbesondere durch kontinuierliche flüssig-flüssig Phasentrennung. Solche Verfahren zur Auftrennung sind dem Fachmann z. B. aus der Kirk-Othmer Encyclopedia of Chemical Technology bekannt.

Die zur destillativen Reingewinnung der einzelnen Produkte, vor allem der gewünschten Propylamine, benötigten Destillationskolonnen können durch den Fachmann mit ihm geläufigen Methoden ausgelegt werden (z. B. Zahl der Trennstufen, Rücklaufverhältnis, etc.).

Besonders bevorzugt ist die Auftrennung des aus dem Reaktor der n-Propanol-Umsetzung (Reaktor 1) resultierenden Reaktionsaustrags in zwei Trennsequenzen durch mehrstufige Destillation, wobei in der ersten Trennsequenz (Trennsequenz 1) zunächst Ammoniak und gegebenenfalls vorhandener Wasserstoff abgetrennt werden und in der zweiten Trennsequenz (Trennsequenz 2) eine Auftrennung in n-Propanol, MPA, DPA, TPA, Wasser und Nebenkomponenten (N K) erfolgt.
Bei dieser Auftrennung des aus dem Reaktor der n-Propanol-Umsetzung (Reaktor 1) resultierenden Reaktionsaustrags durch unvollständige Umsetzung gegebenenfalls anfallendes n-Propanol wird bevorzugt in den Reaktor (Reaktor 1) zurückgeführt.

Das bei dieser Auftrennung anfallende Dipropylamin (DPA) wird, gegebenenfalls je nach Bedarf nach Abzweigung einer Teilmenge in einem Lagertank, in einem separaten Reaktor (Konvertierungsreaktor, Reaktor 2) zur Umsetzung zu Tri-n-propylamin (TPA) in Gegenwart eines kupferhaltigen Heterogen-Katalysators gefahren.
Die Disproportionierung von DPA zu TPA im separaten Reaktor (Reaktor 2), der natürlich auch in zwei oder mehr seriell oder parallel geschaltete Reaktoren aufgeteilt sein kann, wird nach dem oben beschrieben Verfahren durchgeführt.
Zur Aufrechterhaltung der Katalysatoraktivität wird Wasserstoff in den Reaktor (Reaktor 2) gefahren.

Der Ammoniak- und Wasserstoff-haltige Reaktionsaustrag der separaten Umsetzung von DPA zu TPA wird in einer Ausführungsform des erfindungsgemäßen integrierten Verfahrens (Variante 1) mit dem Austrag des Reaktors der n-Propanol-Umsetzung (Reaktor 1) vereinigt und gemeinsam aufgearbeitet, also der Auftrennung des aus dem Reaktor der n-Propanol-Umsetzung (Reaktor 1) resultierenden Reaktionsaustrags, insbesondere der ersten Trennsequenz (Trennsequenz 1) der Auftrennung des aus Reaktor der n-Propanol-Umsetzung (Reaktor 1) resultierenden Reaktionsaustrags, zugeführt.
Ein Verfahrensschema dieser Variante 1 des erfindungsgemäßen integrierten Verfahrens befindet sich in der Anlage (Abbildung 1).

In einer weiteren Ausführungsform des erfindungsgemäßen integrierten Verfahrens (Variante 2) wird aus dem Reaktionsaustrag der separaten Umsetzung von DPA zu TPA zunächst Wasserstoff und Ammoniak abgetrennt (Trennsequenz 3) und bevorzugt jeweils zurückgeführt (Ammoniak in den Reaktor der n-Propanol-Umsetzung (Reaktor 1), Wasserstoff in den Reaktor der n-Propanol-Umsetzung (Reaktor 1) und/oder Reaktor der DPA-Umsetzung (Reaktor 2) und der verbleibende Reaktionsaustrag, enthaltend n-Propylamine, dann der zweiten Trennsequenz (Trennsequenz 2) der Auftrennung des aus dem Reaktor der n-Propanol-Umsetzung (Reaktor 1) resultierenden Reaktionsaustrags zugeführt.
Ein Verfahrensschema dieser Variante 2 des erfindungsgemäßen integrierten Verfahrens befindet sich in der Anlage (Abbildung 2).

Durch die Integration der Disproportionierungsstufe in ein konventionelles n-Propylamine-Herstellverfahren auf Basis n-Propanol können die Austräge aus beiden Reaktionen vorteilhaft gemeinsam aufgearbeitet werden.
Freigesetzter, bei der Aufarbeitung anfallender Ammoniak kann in die Aminierung von n-Propanol (Reaktor 1) zurückgefahren und bei der Aufarbeitung anfallender Wasserstoff kann in den Konvertierungsreaktor (Reaktor 2) und/oder in die Aminierung von n-Propanol (Reaktor 1) zurückgefahren werden.

Alle Druckangaben beziehen sich auf den Absolutdruck.

### Beispiele

### Katalysator ,A S4'

Der Katalysator ,A S4', ein Cu/Co/Ni/gamma-Al₂O₃ - Katalysator wie DE 19 53 263 A (BASF AG) offenbart, wurde durch Tränkung von 4 mm Strängen hergestellt.

Der Katalysator wies die folgende Zusammensetzung vor seiner Behandlung (Aktivierung) mit Wasserstoff auf:
10 Gew.-% CoO, 10 Gew.-% NiO und 4 Gew.-% CuO auf gamma-Al₂O₃.

### Beispiel 1

Das Edukt 100 % DPA wurde am Katalysator ,A S4' in Abwesenheit von NH₃ kontinuierlich disproportioniert. Bei einem Druck von 140 bar, einer Belastung von 0,50 kg/l•h und einer Wasserstoffmenge von 500 Nl/l•h wurde bei 250 °C folgende Zusammensetzung des Reaktionsaustrags bekommen (in Gew.-%): 4 % MPA, 51 % DPA und 45 % TPA.

### Beispiel 2

Das Edukt 100 % DPA wurde am Katalysator ,A S4' in Abwesenheit von NH₃ kontinuierlich disproportioniert. Bei einem Druck von 85 bar, einer Belastung von 0,51 kg/l h und einer Wasserstoffmenge von 400 Nl/l•h wurde bei 220 °C folgende Zusammensetzung des Reaktionsaustrags bekommen (in Gew.-%): 4 % MPA, 47 % DPA und 49 % TPA.

### Beispiel 3

Das Edukt 100 % DPA wurde am Katalysator ,A S4' in Abwesenheit von NH₃ kontinuierlich disproportioniert. Bei einem Druck von 40 bar, einer Belastung von 0,59 kg/l•h und einer Wasserstoffmenge von 405 Nl/l•h wurde bei 215 °C folgende Zusammensetzung des Reaktionsaustrags bekommen (in Gew.-%): 5 % MPA, 50 % DPA und 45 % TPA.

### Beispiel 4

Das Edukt 100 % DPA wurde am Katalysator ,A S4' in Abwesenheit von NH₃ kontinuierlich disproportioniert. Bei einem Druck von 140 bar, einer Belastung von 0,50 kg/l•h und einer Wasserstoffmenge von 600 Nl/l•h wurde bei 250 °C folgende Zusammensetzung des Reaktionsaustrags bekommen (in Gew.-%): 4 % MPA, 50 % DPA und 46 % TPA.

### Beispiel 5

Das Edukt 100 % DPA wurde am Katalysator ,A S4' in Abwesenheit von NH₃ kontinuierlich disproportioniert. Bei einem Druck von 40 bar, einer Belastung von 3,00 kg/l•h und einer Wasserstoffmenge von 200 Nl/l•h wurde bei 240 °C folgende Zusammensetzung des Reaktionsaustrags bekommen (in Gew.-%): 6 % MPA, 51 % DPA und 43 % TPA.

### Beispiel 6

Das Edukt 100 % DPA wurde am Katalysator ,A S4' in Abwesenheit von NH₃ kontinuierlich disproportioniert. Bei einem Druck von 85 bar, einer Belastung von 0,50 kg/l-h und einer Wasserstoffmenge von 200 Nl/l•h wurde bei 200 °C folgende Zusammensetzung des Reaktionsaustrags bekommen (in Gew.-%): 4 % MPA, 57 % DPA und 39 % TPA.

## Patentansprüche

1. Verfahren zur Herstellung von Tri-n-propylamin, **dadurch gekennzeichnet, dass** man Di-n-Propylamin in Gegenwart von Wasserstoff und eines kupferhaltigen Heterogen-Katalysators umsetzt.

2. Verfahren nach Anspruch 1, **dadurch gekennzeichnet, dass** man Di-n-Propylamin in Gegenwart eines kupfer- und nickelhaltigen Heterogen-Katalysators umsetzt.

3. Verfahren nach Anspruch 1, **dadurch gekennzeichnet, dass** man Di-n-Propylamin in Gegenwart eines kupfer- und nickel- und kobalthaltigen Heterogen-Katalysators umsetzt.

4. Verfahren nach Anspruch 1, 2 oder 3, **dadurch gekennzeichnet, dass** der Heterogen-Katalysator Aluminiumoxid und/oder Zirkoniumdioxid als Trägermaterial enthält.

5. Verfahren nach einem der vorhergehenden Ansprüche, **dadurch gekennzeichnet, dass** man kein Ammoniak (NH₃) einsetzt.

6. Verfahren nach einem der vorhergehenden Ansprüche, **dadurch gekennzeichnet, dass** der Heterogen-Katalysator in seiner katalytisch aktiven Masse vor der Behandlung mit Wasserstoff
20 bis 90 Gew.-%, sauerstoffhaltige Verbindungen des Aluminiums, berechnet als Al₂O₃,
1 bis 30 Gew.-% sauerstoffhaltige Verbindungen des Kupfers, berechnet als CuO,
3 bis 30 Gew.-% sauerstoffhaltige Verbindungen des Nickels, berechnet als NiO, und
3 bis 30 Gew.-% sauerstoffhaltige Verbindungen des Kobalts, berechnet als CoO, enthält.

7. Verfahren nach einem der vorhergehenden Ansprüche, **dadurch gekennzeichnet, dass** man die Umsetzung bei einem Absolutdruck im Bereich von 20 bis 150 bar durchführt.

8. Verfahren nach einem der vorhergehenden Ansprüche, **dadurch gekennzeichnet, dass** man die Umsetzung bei einer Temperatur im Bereich von 180 bis 260 °C durchführt.

9. Verfahren nach einem der vorhergehenden Ansprüche, **dadurch gekennzeichnet, dass** man die Umsetzung kontinuierlich durchführt.

10. Verfahren nach einem der vorhergehenden Ansprüche, **dadurch gekennzeichnet, dass** man die Umsetzung bei einer Katalysatorbelastung im Bereich von 0,3 bis 3 kg Di-n-Propylamin / (Liter Katalysator Stunde) durchführt.

11. Verfahren nach einem der vorhergehenden Ansprüche, **dadurch gekennzeichnet, dass** die eingesetzte Wasserstoffmenge im Bereich von 200 bis 2000 Normliter / (Liter Katalysator • Stunde) liegt.

12. Integriertes Verfahren zur Herstellung von Tri-n-propylamin (TPA), **dadurch gekennzeichnet, dass** es die folgenden Operationen umfasst:
I) Umsetzung von n-Propanol mit Ammoniak in einem Reaktor in Gegenwart eines Aminierungskatalysators und gegebenenfalls Wasserstoff zu einem Gemisch von Mono-n-propylamin, Di-n-propylamin (DPA) und TPA,
II) Abtrennung von unumgesetztem Ammoniak, unumgesetztem n-Propanol und gegebenenfalls Wasserstoff aus dem Reaktionsaustrag und Rückführung zumindest des Ammoniaks und Propanols in den Reaktor in I), sowie destillative Auftrennung des n-Propylamingemischs und Abtrennung des TPAs,
III) Umsetzung des in II) bei der destillativen Auftrennung anfallenden DPAs in einem Reaktor nach einem Verfahren gemäß einem der Ansprüche 1 bis 11 zu TPA und
IV) Zuführung des Reaktoraustrags aus III) in die Operation II).

13. Integriertes Verfahren nach dem vorhergehenden Anspruch, **dadurch gekennzeichnet, dass** vor Operation IV) aus dem Reaktoraustrag aus III) zunächst Ammoniak und Wasserstoff abgetrennt und zumindest der Ammoniak in den Reaktor in I) zurückgeführt wird.

## Claims

1. A process for preparing tri-n-propylamine, wherein di-n-propylamine is reacted in the presence of hydrogen and a copper-comprising heterogeneous catalyst.

2. The process according to claim 1, wherein di-n-propylamine is reacted in the presence of a copper- and nickel-comprising heterogeneous catalyst.

3. The process according to claim 1, wherein di-n-propylamine is reacted in the presence of a copper- and nickel- and cobalt-comprising heterogeneous catalyst.

4. The process according to claim 1, 2 or 3, wherein the heterogeneous catalyst comprises aluminum oxide and/or zirconium dioxide as support material.

5. The process according to any of the preceding claims, wherein no ammonia (NH₃) is used.

6. The process according to any of the preceding claims, wherein the catalytically active composition of the heterogeneous catalyst before treatment with hydrogen comprises from 20 to 90% by weight of oxygen-comprising compounds of aluminum, calculated as Al₂O₃,
from 1 to 30% by weight of oxygen-comprising compounds of copper, calculated as CuO,
from 3 to 30% by weight of oxygen-comprising compounds of nickel, calculated as NiO, and
from 3 to 30% by weight of oxygen-comprising compounds of cobalt, calculated as CoO.

7. The process according to any of the preceding claims, wherein the reaction is carried out at an absolute pressure in the range from 20 to 150 bar.

8. The process according to any of the preceding claims, wherein the reaction is carried out at a temperature in the range from 180 to 260°C.

9. The process according to any of the preceding claims, wherein the reaction is carried out continuously.

10. The process according to any of the preceding claims, wherein the reaction is carried out at a space velocity over the catalyst in the range from 0.3 to 3 kg of di-n-propylamine/(liters of catalyst • hour).

11. The process according to any of the preceding claims, wherein the amount of hydrogen used is in the range from 200 to 2000 standard liters/(liters of catalyst hour).

12. An integrated process for preparing tri-n-propylamine (TPA), which comprises the following operations:
I) reaction of n-propanol with ammonia in a reactor in the presence of an amination catalyst and optionally hydrogen to form a mixture of mono-n-propylamine, di-n-propylamine (DPA) and TPA,
II) separation of unreacted ammonia, unreacted n-propanol and possibly hydrogen from the reaction product mixture and recirculation of at least the ammonia and propanol to the reactor in I) and also separation of the n-propylamine mixture by distillation and isolation of the TPA,
III) reaction of the DPA obtained in the separation by distillation in II) in a reactor by a process according to any of claims 1 to 11 to form TPA and
IV) feeding of the reactor output from III) to operation II).

13. The integrated process according to the preceding claim, wherein ammonia and hydrogen are firstly separated off from the reactor output from III) before operation IV) and at least the ammonia is recirculated to the reactor in I).

## Revendications

1. Procédé pour la préparation de tri-n-propylamine, **caractérisé en ce qu'**on transforme de la di-n-propylamine en présence d'hydrogène et d'un catalyseur hétérogène contenant du cuivre.

2. Procédé selon la revendication 1, **caractérisé en ce qu'**on transforme de la di-n-propylamine en présence d'un catalyseur hétérogène contenant du cuivre et du nickel.

3. Procédé selon la revendication 1, **caractérisé en ce qu'**on transforme de la di-n-propylamine en présence d'un catalyseur hétérogène contenant du cuivre, du nickel et du cobalt.

4. Procédé selon la revendication 1, 2 ou 3, **caractérisé en ce que** le catalyseur hétérogène contient de l'oxyde d'aluminium et/ou du dioxyde de zirconium comme matériau support.

5. Procédé selon l'une quelconque des revendications précédentes, **caractérisé en ce qu'**on n'utilise pas d'ammoniac (NH₃) .

6. Procédé selon l'une quelconque des revendications précédentes, **caractérisé en ce que** le catalyseur hétérogène contient, dans sa masse catalytiquement active, avant le traitement avec l'hydrogène
20 à 90% en poids, de composés oxygénés de l'aluminium, calculés sous forme d'Al₂O₃,
1 à 30% en poids de composés oxygénés du cuivre, calculés sous forme de CuO,
3 à 30% en poids de composés oxygénés du nickel, calculés sous forme de NiO, et
3 à 30% en poids de composés oxygénés du cobalt, calculés sous forme de CoO.

7. Procédé selon l'une quelconque des revendications précédentes, **caractérisé en ce qu'**on réalise la transformation à une pression absolue dans la plage de 20 à 150 bars.

8. Procédé selon l'une quelconque des revendications précédentes, **caractérisé en ce qu'**on réalise la transformation à une température dans la plage de 180 à 260°C.

9. Procédé selon l'une quelconque des revendications précédentes, **caractérisé en ce qu'**on réalise la transformation en continu.

10. Procédé selon l'une quelconque des revendications précédentes, **caractérisé en ce qu'**on réalise la transformation à une charge du catalyseur dans la plage de 0,3 à 3 kg de di-n-propylamine/(litre de catalyseur.heure).

11. Procédé selon l'une quelconque des revendications précédentes, **caractérisé en ce que** la quantité d'hydrogène utilisée se situe dans la plage de 200 à 2000 litres normaux/(litre de catalyseur.heure).

12. Procédé intégré pour la préparation de tri-n-propylamine (TPA), **caractérisé en ce qu'**il comprend les opérations suivantes :
I) transformation de n-propanol avec de l'ammoniac dans un réacteur en présence d'un catalyseur d'amination et le cas échéant de l'hydrogène en un mélange de mono-n-propylamine, de di-n-propylamine (DPA) et de TPA,
II) séparation de l'ammoniac non transformé, du n-propanol non transformé et éventuellement de l'hydrogène du produit évacué de la réaction et recyclage au moins de l'ammoniac et du propanol dans le réacteur dans I), ainsi que séparation par distillation du mélange de n-propylamine et séparation de la TPA,
III) transformation de la DPA obtenue lors de la séparation par distillation dans l'étape II) dans un réacteur selon un procédé selon l'une quelconque des revendications 1 à 11 en TPA et
IV) alimentation du produit évacué du réacteur de III) dans l'opération II).

13. Procédé intégré selon la revendication précédente, **caractérisé en ce qu'**on sépare, avant l'opération IV), du produit évacué de la réaction de III), d'abord l'ammoniac et l'hydrogène et on recycle au moins l'ammoniac dans le réacteur dans I).
